# EUROPEAN PATENT APPLICATION

(11) **EP 0 976 409 A1**
(43) Date of publication of application: **02.02.2000**
(21) Application number: 98950350.3
(22) Date of filing: 22.10.1998
(51) Int. Cl.: A61L 2/18, G02C 13/00

(54) **TREATING CONTAINER OF CONTACT LENS**

(30) Priority: 24.10.1997 JP 29293897
(71) Applicant: TOMEY CORPORATION, Nagoya-shi, Aichi 451-0051 (JP)
(72) Inventor: NAKAGAWA, Makoto, Higashino-cho Kasugai-shi Aichi 486-0817 (JP); NAGAO, Tomio, Higashino-cho Kasugai-shi Aichi 486-0817 (JP); MIYAWAKI, Takeshi Tomey Corporation, Kasugai-shi Aichi 486-0817 (JP); MATSUMOTO, Satoru Tomey Corporation, Nagoya-shi Aichi 451-0053 (JP); SUGUIRA, Atsuko Tomey Corporation, Nagoya-shi Aichi 451-0053 (JP)
(74) Representative: Paget, Hugh Charles Edward
(86) International application number: JP9804792
(87) International publication number: WO9921594

(57) **Abstract**

It is an object of the present invention to provide a contact lens treating container used for effecting a suitable treatment of the contact lens by mixing two different treating agents, the two treating agents being mixed with each other in a fluid-tightly sealed condition.

The treating container is constructed with a container body 12 for accommodating a liquid first treating agent 40 in which a contact lens 38 is immersed, and retaining means 32 disposed in a fluid-tight space defined upon closure of the container body 12, for retaining a second treating agent 42, such that the second treating agent 42 is inhibited from freely mixing with the first treating agent 40 while the container body 12 is kept stationary. The first treating agent 40 in the container body 12 and the second treating agent 42 retained by the retaining means 32 are freely mixed with each other by shaking the container body 12 which is fluid-tightly closed.

## Description

### Technical Field

The present invention relates to an improved structure of a treating container for a contact lens, which is used for effecting a suitable treatment on the contact lens accommodated therein.

### Background Art

For assuring a safe and comfortable wearing condition of a contact lens, the contact lens is subjected to various treatments such as a disinfecting treatment and a cleaning treatment on a regular basis. That is, the contact lens is disinfected and cleaned every day or at regular intervals, whereby various microorganisms such as bacteria and fungi adhering to the surfaces of the contact lens are sterilized, and the protein and lipid deposits are removed from the lens surfaces, so as to avoid various eye infectious diseases and eye troubles.

Among various treatments effected on the contact lens, the following two methods are well known for disinfecting the contact lens: a thermal disinfecting method in which the contact lens is boiled for disinfection; and a chemical disinfecting method in which the contact lens is disinfected by contact with various kinds of chemical disinfectants. In recent years, the chemical disinfecting method has been employed more often than the thermal disinfecting method since the chemical disinfecting method does not use any special apparatus such as boiling and disinfecting apparatus as required in the thermal disinfecting method, and assures a simplified disinfecting procedure, so that the contact lens is disinfected at a relatively low cost. In the chemical disinfecting method, a technique which uses an iodine disinfectant or germicide as a disinfecting agent has been attracting a great attention. The iodine disinfectant exhibits excellent antimicrobial activity and safety to the eyes of the contact lens user.

The iodine disinfectant described above, however, has a drawback that the iodine included therein is easily adsorbed on the contact lens, whereby the contact lens is colored in amber or reddish purple peculiar to the color of the iodine after a relatively long period of contact with the iodine disinfectant.

In view of this, when the contact lens is disinfected by using the iodine disinfectant, the contact lens is immersed in the iodine disinfectant, and subsequently a suitable reducing agent is added to and mixed with the iodine disinfectant. According to this procedure, the iodine included in the iodine disinfectant is reduced by the reducing agent immediately after the disinfection of the contact lens is completed. Though the iodine disinfectant in which the iodine has been reduced no more exhibits the disinfecting activity, the disinfectant is made colorless and transparent due to the reduction of the iodine. Thus, the disinfected contact lens is protected from being colored by the iodine.

When the contact lens is disinfected by using the iodine disinfectant, there has been conventionally used a treating container including a container body which accommodates the contact lens while it is immersed in the iodine disinfectant, and a cap member to be assembled with the container body. In the conventional treating container, the cap member needs to be removed from the container body at least when the reducing agent is added to and mixed with the iodine disinfectant. In this case, the inside of the container body, and the iodine disinfectant and the contact lens accommodated therein are brought into contact with the external air.

In disinfecting the contact lens by using the treating container as described above, the bacteria present in the atmosphere inevitably enter the container body upon addition of the reducing agent for mixture with the iodine disinfectant, even if the container body is fluid-tightly sealed by the cap member immediately after the reducing agent is added to and mixed with the iodine disinfectant. In this case, it is difficult to inhibit proliferation of the bacteria which have entered the container body, since the disinfectant has lost its disinfecting activity as a result of the reduction of the iodine. Accordingly, the disinfected contact lens may be contaminated again with the bacteria proliferated in the container body.

In effecting an additional treatment on the contact lens after the contact lens is disinfected by a disinfectant which is different from the iodine disinfectant and which does not cause the coloring of the contact lens, the contact lens is initially immersed in the disinfectant accommodated in the above-described treating container with the cap member, and subsequently a suitable treating agent for the desired treatment is added to and mixed with the disinfectant. In this case, too, the bacteria in the atmosphere inevitably get into the container body upon addition of the treating agent for mixture with the disinfectant, causing a problem of insufficient disinfection of the contact lens.

Thus, when the contact lens is treated with the two different treating agents by using the conventional treating container as described above, the contact lens is immersed in one of the two treating agents in a liquid form, and the other treating agent is added to and mixed with the above-indicated one treating agent, so that the contact lens is treated as desired. In this procedure, however, the cap member is kept removed from the container body while the other treating agent is added to and mixed with the one treating agent. In this case, the inside of the container body, and the contact lens and the two treating agents accommodated therein are inevitably brought into contact with the external air, causing various troubles as described above.

### Disclosure of the Invention

The present invention was developed in the light of the above situations. It is therefore an object of the present invention to provide a treating container for a contact lens used in treating the contact lens with two different treating agents by immersing the contact lens in one of the two treating agents in a liquid form and adding the other treating agent for mixture with the above-indicated one treating agent, wherein the other treating agent is added to and mixed with the one treating agent with the treating container being fluid-tightly sealed, so as to prevent the inside of the container and the contact lens and the two treating agents accommodated therein from contacting the external air, to thereby avoid various troubles which would be otherwise caused by contact with the external air, and wherein the two treating agents are effectively mixed with each other in a relatively short period of time.

The above object may be attained according to the invention which provides a treating container for a contact lens characterized by comprising: a container body accommodating a liquid first treating agent in which the contact lens to be treated is immersed, and having an upper opening through which the contact lens is brought into and out of the container body; a cap member which fluid-tightly closes the opening of the container body; and retaining means which is disposed in a fluid-tight space defined upon closure of the opening of the container body by the cap member, the retaining means retaining a second treating agent for treating the contact lens, such that the second treating agent is inhibited from freely mixing with the first treating agent while the container body is kept stationary, and such that the first treating agent in the container body and the second treating agent retained by the retaining means can be freely mixed with each other when the container body is shaken while the opening is closed by the cap member.

In the present contact lens treating container constructed as described above, the liquid first treating agent is accommodated in the container body with the contact lens being immersed therein, while the second treating agent is retained by the retaining means located in the fluid-tight space in the container body when the container body is closed by the cap member. In this arrangement, the first and second liquid agents are inhibited from mixing with each other while the container body is kept stationary, and the two treating agents can be freely mixed with each other by shaking the container body while its opening is fluid-tightly closed by the cap member. Accordingly, there is no need to remove and attach the cap member from and to the container body when the second treating agent is added for mixture with the first treating agent in which the contact lens is immersed, whereby the contact lens and the first and second treating agents and the inside of the container body are advantageously inhibited from contacting the external air.

In the thus constructed contact lens treating container of the present invention, the contact lens can be sufficiently and smoothly treated by using the two different treating agents, without suffering from various troubles as experienced in the conventional container by exposure of the inside of the container body and the two treating agents and the contact lens accommodated therein to the external air when one of the two treating agents which is a liquid and in which the contact lens is immersed is mixed with the other treating agent added thereto. In this arrangement, it is not necessary to attach and remove the cap member to and from the container body when the other agent is added to and mixed with the above-described one liquid agent, facilitating the procedure of mixing those two treating agents.

In the present contact lens treating container described above, one of an iodine disinfectant and a reducing agent is accommodated in the container body with the contact lens being immersed therein, while the other of the iodine disinfectant and the reducing agent is retained by the retaining means. In this state, the container body which is fluid-tightly closed by the cap member is shaken, so that the reducing agent and the iodine disinfectant are mixed with each other, such that the contact lens, the reducing agent and the iodine disinfectant are prevented from being exposed to the external air. Accordingly, the contact lens can be sufficiently disinfected while it is effectively prevented from being colored with the iodine. Further, the present contact lens treating container can be used when the contact lens is disinfected with a disinfectant other than the iodine disinfectant. Namely, the selected disinfectant is accommodated in the container body with the contact lens being immersed therein while a suitable treating agent is retained by the retaining means. In this state, the container body which is fluid-tightly closed by the cap member is shaken, so that the disinfectant and the treating agent are mixed with each other, such that the contact lens, the disinfectant and treating agent are prevented from being exposed to the external air. Accordingly, the contact lens can be treated as desired while it is effectively disinfected.

In one preferred form of the contact lens treating container of the present invention, the retaining means is constituted by at least one projection which protrudes from an inner surface of the cap member so as to form a minute gap, the second treating agent being retained in the minute gap against gravity. According to this arrangement, the retaining means can be easily formed by simply providing the projection which protrudes from the inner surface of the cap member so as to form the minute gap, effectively preventing the structure of the treating container from being complicated due to the provision of the retaining means.

The above-described projection as the retaining means preferably comprises a plurality of concentric annular projecting ribs which protrude from the inner surface of the cap member, the minute gap being formed between adjacent ones of the plurality of projecting ribs.

In this arrangement, the amount of the second treating agent retained by the retaining means can be easily adjusted by changing the number of the projecting ribs formed on the inner surface of the cap member, in other words, the number of the minute gaps formed between the adjacent ones of the plurality of projecting ribs. The annular projecting ribs of this arrangement are effective to retain the second treating agent with high stability, unlike projecting ribs formed in parallel with each other wherein the minute gaps defined between adjacent ones of the parallel projecting ribs are open at the opposite ends of the projecting ribs.

The present invention is also characterized by providing a treating container for a contact lens which comprises a container body accommodating a liquid first treating agent in which the contact lens to be treated is immersed, and having an upper opening through which the contact lens is brought into and out of the container body; a cap member which fluid-tightly closes the opening of the container body; and an accommodating structure disposed in a fluid-tight space defined upon closure of the opening of the container body by the cap member, and having an accommodating chamber for accommodating therein a second treating agent for treating the contact lens, and a communication hole which permits a flow of the first treating agent into the accommodating chamber, the first and second treating agents being inhibited from freely mixing with each other while the container body is kept stationary, and such that the first treating agent in the container body and the second treating agent in the accommodating structure can be fed through the communication hole when the container body is shaken while the opening is closed by the cap member.

In the present contact lens treating container constructed as described above, the liquid first treating agent is accommodated in the container body with the contact lens being immersed therein while the second treating agent is accommodated in the accommodating chamber formed in the accommodating structure, which permits a flow of the first treating agent thereinto through the communication hole while the container body is closed by the cap member. The first and second treating agents are prevented from mixing with each other while the container body is kept stationary. By shaking the container body which is fluid-tightly closed by the cap member, the first and second treating agents can be fed through the communication hole of the accommodating structure, so that the two treating agents are mixed with each other. According to this arrangement, the second treating agent can be added to and mixed with the first treating agent without exposure of the first and second treating agents, the contact lens and the inside of the container body to the external air.

Accordingly, the present contact lens treating container constructed as described above is free from the troubles caused by the exposure to the air, so that the contact lens can be effectively and easily treated with the two treating agents.

In one preferred form of the above-described contact lens treating container, the accommodating structure is disposed in the fluid-tight space with a portion thereof being immersed in the first treating agent, the first treating agent flowing into the accommodating structure through the communication hole which is formed in the portion of the accommodating structure immersed in the first treating agent, the accommodating chamber of the accommodating structure being defined by a partition wall whose upper end is located above a level of the first treating agent which has flowed into the accommodating structure, so that the first treating agent is inhibited from flowing into the accommodating chamber while the container body is kept stationary.

The present invention also provides a treating container for a contact lens, characterized by comprising: a container body accommodating a liquid first treating agent in which the contact lens to be treated is immersed, and having an upper opening through which the contact lens is brought into and out of the container body; a cap member which fluid-tightly closes the opening of the container body; and an accommodating structure disposed in a fluid-tight space defined upon closure of the opening of the container body by the cap member, and having an accommodating chamber for accommodating therein a second treating agent for treating the contact lens, and a window which is open to a portion of the fluid-tight space which is located above a level of the first treating agent accommodated in the container body, and such that the first treating agent in the container body and the second treating agent in the accommodating structure can be mixed with each other through the window when the container body is shaken while the opening is closed by the cap member.

In the thus constructed treating container, too, the first and second treating agents are mixed with each other without attaching and removing the cap member to and from the container body, and without exposing the first and second treating agents, the contact lens, and the inside of the container body to the external air.

Accordingly, the present contact lens treating container constructed as described above is free from the troubles caused by the exposure to the air, so that the contact lens can be effectively and easily treated with the two treating agents.

In one advantageous form of the above-described contact lens treating containers, the accommodating structure is fixed to an inner surface of the cap member which defines the fluid-tight space, the accommodating structure being disposed in the fluid-tight space when the container body is closed by the cap member. This arrangement is effective to prevent the first treating agent in the container body and the second treating agent in the accommodating structure from erroneously mixing with each other before the container body is closed by the cap member. Accordingly, the contact lens can be treated as desired with the first and second treating agents which are mixed with each other without any handling mistake.

In another preferred form of the the above-described contact lens treating containers, the accommodation structure is a member separate from the cap member which defines the enclosed space, and has a flange which is sandwiched by and between the cap member and the container body, the accommodating structure being disposed in the fluid-tight space when the container body is closed by the cap member, with the flange being sandwiched by and between the container body and the cap member. In this arrangement, the cap member need not have an inlet through which the treating agent is introduced into the accommodating structure. Accordingly, the treating container does not require a closure member for closing the inlet, resulting in a simplified structure.

In another advantageous form of the above-described contact lens treating containers, the container body includes an accommodating chamber having one opening and storing a pair of contact lenses for a left and a right eye, the pair of contact lenses being brought into and out of the accommodating chamber through the opening. Since this arrangement requires a single cap member for closing the opening of the container body, the structures of the treating containers can be simplified.

In another advantageous form of the above-described contact lens treating containers, one of the first and second treating agents is an iodine disinfectant, while the other is a reducing agent which makes the iodine disinfectant colorless and transparent when the reducing agent is mixed with the iodine disinfectant. According to this arrangement, the reducing agent and the iodine disinfectant are mixed with each other by simply shaking the container body which is closed by the cap member, such that the contact lens, the reducing agent and the iodine disinfectant are prevented from being exposed to the external air. Therefore, the contact lens can be sufficiently disinfected while it is prevented from being colored with the iodine.

When the iodine disinfectant and the reducing agent are used as one and the other of the first and second treating agents, the container body is preferably formed of a transparent resin material. According to this arrangement, the iodine disinfectant can be effectively made colorless and transparent upon mixture with the reducing agent. Further, it is possible to visually inspect the reduction of the iodine, and to confirm termination of the reduction of the iodine.

### Brief Description of Drawings

Fig. 1 is an exploded view in longitudinal cross section, showing a contact lens treating container constructed according to one embodiment of the present invention;
Fig. 2 is an enlarged view in longitudinal cross section, showing a principal part of the treating container of Fig. 1, when it is used for treating the contact lens;
Fig. 3 is an enlarged view showing a principal part of a contact lens treating container according to another embodiment of the invention;
Fig. 4 is is an enlarged view showing a principal part of a contact lens treating container according to still another embodiment of the invention;
Fig. 5 is a view corresponding to that of Fig. 2, showing a contact lens treating container according to yet another embodiment of the invention;
Fig. 6 is a top plan view of an accommodating structure used in the contact lens treating container of Fig. 5;
Fig. 7 is a view corresponding to that of Fig. 2, showing a contact lens treating container according to a further embodiment of the invention;
Fig. 8 is a cross sectional view taken along line VIII-VIII in Fig. 7;
Fig. 9 is a view corresponding to that of Fig. 2, showing a contact lens treating container according to another embodiment of the invention;
Fig. 10 is a plan view showing the inner surface of a cap member used in the contact lens treating container of Fig. 9;
Fig. 11 is an elevational view in longitudinal cross section of a cap member used in a contact lens treating container according to still another embodiment of the invention;
Fig. 12 is a view corresponding to that of Fig. 11, showing a cap member used in a contact lens treating container according to yet another embodiment of the invention;
Fig. 13 is a view in longitudinal cross section showing a contact lens treating container according to a further embodiment of the invention when the container is used;
Fig. 14 is a view corresponding to that of Fig. 13, showing a contact lens treating container according to yet another embodiment of the invention; and
Fig. 15 is a view corresponding to that of Fig. 13, showing a contact lens treating container according to a further embodiment of the invention.

### Best mode for carrying out the invention

To further clarify the present invention, there will be described in detail specific structures of the contact lens treating containers constructed according to some embodiments of the present invention referring to the drawings.

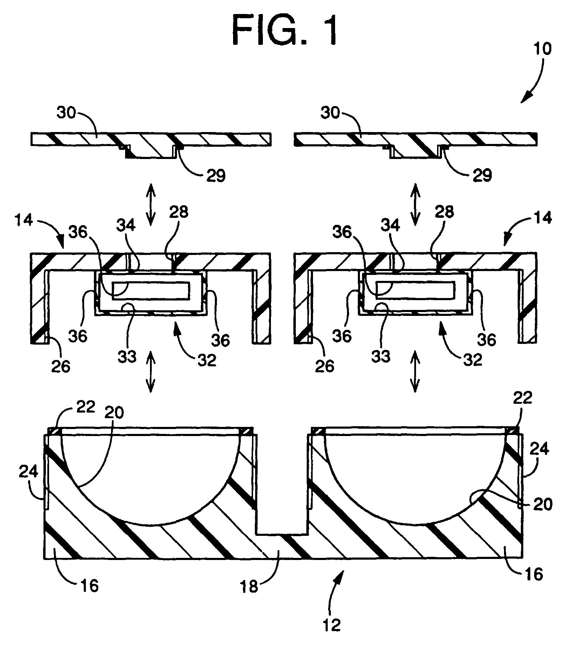

As shown in Fig. 1 which is an exploded view in longitudinal cross section of a contact lens treating container 10 constructed according to one embodiment of the present invention, the treating container 10 includes a container body 12 and a pair of cap members 14, 14.

Described more specifically, the container body 12 of the treating container 10 consists of a pair of receptacles 16, 16 and a connecting portion 18 which connects the two receptacles 16, 16 to each other. Each receptacle 16 has a recess 20 having an upper opening and a semi-spherical bottom surface. The recess 20 is dimensioned such that the diameter of its opening is large enough to permit the contact lens not shown to be easily put into or taken out of the recess, and such that the depth of the recess 20 is large enough to accommodate the contact lens together with a suitable treating agent in which the contact lens is immersed.

On the upper end face of each receptacle 16, there is bonded an annular sealing rubber 22 along the periphery of the opening of the recess 20. The receptacle 16 has an externally threaded portion 24 formed on the outer circumferential surface of its axially upper portion. The thus constructed pair of receptacles 16, 16 are arranged side by side with their lower portions being integrally connected to each other by the plate-like connecting portion 18, so that the container body 12 has an integral structure consisting of the two receptacles 16, 16 and the connecting member 18.

In the present embodiment, the container body 12 is preferably formed of a transparent resin material which permits transmission of ordinary indoor light, preferably not smaller than 40% of a light having a wavelength of 350-900nm, more preferably not smaller than 60% of such a light. When an iodine-containing oxidizing agent used as the second treating agent is mixed with a reducing agent which contains ethylenediamine tetraacetic acid (EDTA) or its salt and which is used as the first treating agent, in the container body 12 in the manner described below, the oxidizing agent and the reducing agents are sufficiently exposed to the light transmitted through the container body 12 formed of the material as described above, whereby the iodine in the oxidizing agent is effectively made colorless and transparent by the EDTA.

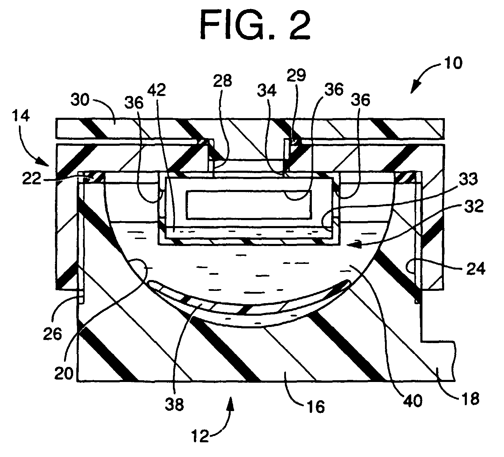

Each of the cap members 14, 14 is a cylindrical member closed at one of its opposite ends, and has a small height or axial dimension. The bottom wall of the cap member 14 has a size large enough to close the opening of the recess 20 of the receptacle 16 of the container body 12. The cap member 14 has a through-hole 28 formed through the thickness of the central part of the bottom wall. This through-hole 28 is fluid-tightly closed by a plug 30 having a seal packing 29. This plug 30 removably attached to the cap member 14 is formed of a material similar to that of the container body 12 and the cap member 14. The cap member 14 has an internally threaded portion 26 which is formed on the inner circumferential surface of its cylindrical portion, for engaging the externally threaded portion 24 of the receptacle 16 of the container body 12. According to this arrangement, the cap member 14 is removably attached to the receptacle 16 of the container body 12 to fluid-tightly close the opening of the recess 20. Upon assembling of the cap member 14 with the receptacle 16 of the container body 12, the recess 20 is fluid-tightly closed, to thereby form a fluid-tight space (as shown in Fig. 2). The cap member 14 is also formed of the transparent resin material similar to that of the container body 12, so that the iodine in the oxidizing agent can be effectively made colorless and transparent by the EDTA upon mixing of the two treating agents in the container body 12.

In the present treating container, an accommodating structure 32 is disposed on the inner surface of the bottom wall of each cap member 14. This accommodating structure 32 has a generally rectangular box-like configuration having a relatively small wall thickness. The inner space of the accommodating structure 32 functions as an accommodating chamber 33 having a size large enough to accommodate a suitable treating agent (not shown) in a liquid or solid form. The accommodating structure 32 has a communication hole 34 formed through the thickness of its upper wall. This communication hole 34 has substantially the same size as the through-hole 28 of the cap member 14. The accommodating structure 32 is fixed by an adhesive, for instance, at its outer surface of the upper wall to the inner surface of the bottom wall of the cap member 14, such that the communication hole 34 is aligned with the through-hole 28 of the cap member 14, in other words, such that the accommodating chamber 33 is open upwards through the communication hole 34 and the through-hole 28.

The accommodating structure 32 has four rectangular windows 36 respectively formed through its four side walls. Each window 36 has a size large enough to permit the liquid or solid treating agent (not shown) in the accommodating structure 32 to mix with the treating agent in the recess 20 of the container body 12. Each of the four windows 36 is formed in the accommodating structure 32 such that the window 36 is located at an upper portion of the fluid-tight space which is partially defined by the recess 20 upon assembling of the cap member 14 with the receptacle 16, in other words, upon closure of the recess 20 by the cap member 14.

In the treating container 10 of the present embodiment, a contact lens 38 is immersed in a reducing agent 40 accommodated in the recess 20 of the container body 12. The reducing agent 40, which is used as a first treating agent, contains ethylenediamine tetraacetic acid (EDTA) or its salt and suitably selected pH buffer and tonicity adjusting agent. In the accommodating chamber 33 of the accommodating structure 32 which is fixed to and retained on the inner surface of the cap member 14, there is accommodated an oxidizing agent 42 as a second treating agent, which contains iodine (e.g., iodine-associated or iodine-complex polymer, I₂). The cap member 14 is assembled with the container body 12 so as to fluid-tightly close the recess 20, so that the lower portion of the accommodating structure 32 in which the oxidizing agent 42 is accommodated is submerged in the reducing agent 40 accommodated in the recess 20. In this state, the four windows 36 of the accommodating structure 32 are located above the liquid surface of the reducing agent 40, so that the windows 36 are open to the fluid-tight space. The oxidizing agent 42 is introduced into the accommodating chamber 33 of the accommodating structure 32 through the through-hole 28 of the cap member 14 and the communication hole 34 of the accommodating structure 32, with the plug 30 being removed from the cap member 14.

The treating container 10 is oscillated or shaken frontwards and rearwards, leftwards and rightwards, or upwards and downwards, with the recess 20 of the container body 14 being fluid-tightly closed by the cap member 14 shown in Fig. 2, so that the oxidizing agent 42 in the accommodating chamber 33 flows therefrom into the recess 20 through the four windows 36, whereby the oxidizing agent 42 is mixed with the reducing agent 40 in the recess 20. At the same time, the reducing agent 40 in the recess 20 flows into the accommodating chamber 33 through the four windows 36, so that the reducing agent 40 is mixed with the oxidizing agent 42 therein, and the thus mixed treating agents in the chamber 33 flow back to the recess 20 through the four windows 36, and thereby mix with the reducing agent 40.

In the treating container 10 according to the present embodiment, the reducing agent 40 and the oxidizing agent 42 are accommodated in the container body 12 and the chamber 33 of the accommodating structure 32, respectively. In this arrangement, by simply shaking the container body 12 which is fluid-tightly closed by the cap member 14, the oxidizing agent 42 and the reducing agent 40 are easily mixed with each other, so that the contact lens immersed in the reducing agent 40 is brought into contact with the oxidizing agent 42. Since the EDTA in the reducing agent 40 used in this embodiment has a relatively weak reducing activity, the iodine in the oxidizing agent 42 is gradually reduced.

By using the treating container 10 constructed as described above, the contact lens 38 immersed in the reducing agent 40 is brought into contact with the iodine for disinfection, such that the contact lens 38, the reducing agent 40 and the oxidizing agent 42 are prevented from being exposed to the external air, while the iodine is reduced by the EDTA in the reducing agent 40, whereby the iodine disinfectant is made colorless and transparent.

Unlike the conventional treating container, the treating container 10 of the present embodiment prevents the oxidizing agent 42, the reducing agent 40 and the contact lens 38 from contacting the external air after the oxidizing agent 42 has lost its disinfecting activity due to the reduction of the iodine. Accordingly, the contact lens 38 is effectively prevented from being contaminated with the bacteria after it has been disinfected by the oxidizing agent 42, so that the contact lens 38 can be sufficiently disinfected while it is prevented from being colored.

In the present treating container 10, the accommodating structure 32, which accommodates the oxidizing agent 42 to be mixed with the reducing agent 40 in the container body, is fixed to the cap member 14 when the container body 12 is fluid-tightly closed by the cap member 14. In this arrangement, the reducing agent 40 and the oxidizing agent 42 are not erroneously mixed with each other before the cap member 14 is assembled with the container body 12. Accordingly, the contact lens can be treated as desired with the two treating agents 40, 42 without any handling mistake.

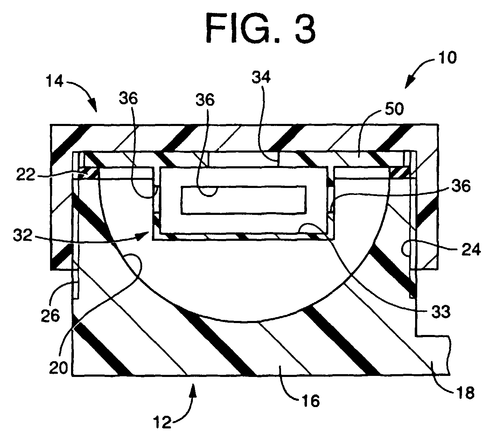

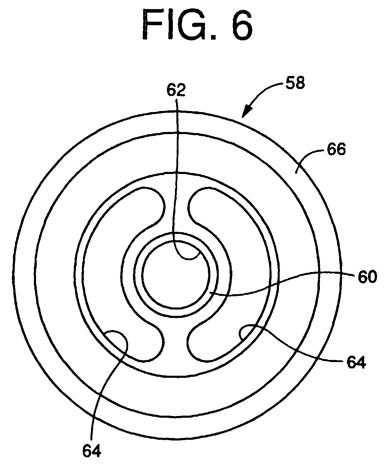

It is to be understood that the structure of the contact lens treating container of the present invention is not limited to that of the above embodiment, but may be otherwise embodied without departing from the scope of the present invention. Some other embodiments will be described by referring to Figs. 3-15. In the embodiments of Figs. 3-15, the same reference numerals as used in the above first embodiment are used to identify the corresponding components, and its detailed explanation is dispensed with. In each of the treating containers shown in Figs. 3-5 and Figs 7-9, only one receptacle of the container body and only one cap member to be attached thereto are shown.

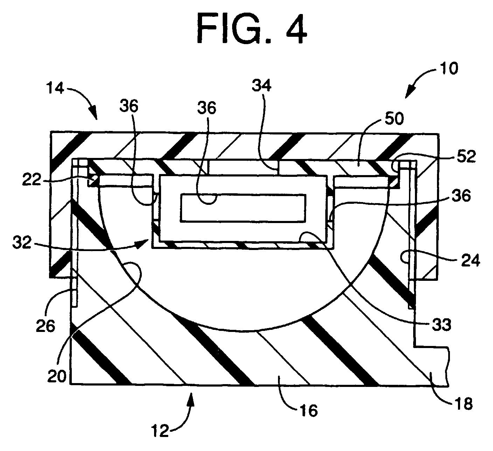

Referring to Figs. 3 and 4, there is shown a treating container having an accommodating structure different from that in the above first embodiment.

In the treating container of Fig. 3, the accommodating structure 32 is a member separate from the cap member 14. The accommodating structure 32 has a flange 50 which extends radially outwardly from the outer peripheral portion of its upper wall. The accommodating structure 32 is disposed in the fluid-tight space defined upon closure of the container body 12 by the cap member 14, such that the flange 50 is sandwiched by and between the cap member 14 and the upper end face of the receptacle 16 of the container body 12.

In the present treating container wherein the accommodating structure 32 is a member separate from the cap member 14, the cap member 14 need not have an inlet (corresponding to the through-hole 28 in the above first embodiment) through which the treating agent is introduced into the accommodating structure 32. It is, therefore, not necessary to provide a member for closing the inlet (corresponding to the plug 30 in the above first embodiment). Accordingly, the number of the required components can be reduced, resulting in a simplified structure of the treating container.

As is apparent from Figs. 3 and 4, the accommodating structure 32 shown in Fig. 3 is superposed on the upper end face of the receptacle 16 of the container body 12 with the sealing rubber 22 interposed therebetween, while the accommodating structure 32 shown in Fig. 4 is superposed on the receptacle 16 such that the outer peripheral portion of the flange 50 is fitted in a shoulder portion 52 formed on the upper end face of the receptacle 16 of the container body 12, via the sealing rubber 22. In the treating container of Fig. 4, the accommodating structure 32 is easily positioned relative to the receptacle 16 of the container body 12 by engagement of the flange 50 with the shoulder portion 52.

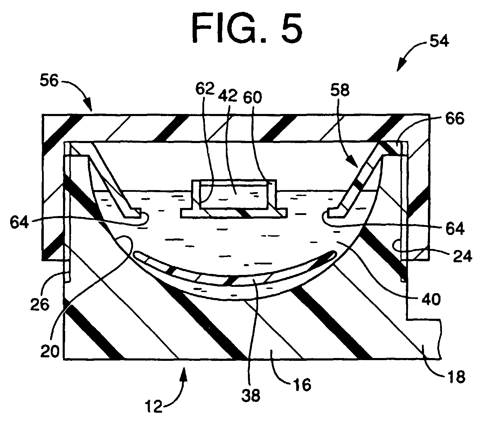

Referring next to Fig. 5, there is shown a treating container 54 constructed according to another embodiment of the invention, wherein the accommodating structure and the cap member are different from those of the above embodiments. The treating container 54 of this embodiment includes a container body 12, a pair of cap members 56, 56, and a pair of accommodating structures 58, 58, which are formed of a transparent resin material. (In Fig. 5, only one of the cap members and one of the accommodating structures are shown.) As in the above first embodiment, the container body 12 of this embodiment has an integral structure consisting of a pair of receptacles 16, 16 having respective recesses 20, 20 for accommodating therein the reducing agent 40 as the first treating agent and the contact lens 38, and a connecting portion 18 which connects the two receptacles 16, 16 to each other. (In Fig. 5, only one of the receptacles is shown.) The cap member 56 is a cylindrical member closed at one of its opposite ends and having a relatively small axial dimension. The cap member 56 is dimensioned such that its bottom wall has a size large enough to close the opening of the recess 20 of the receptacle 16 of the container body 12. The cap member 56 has an internally threaded portion 26 formed on the inner circumferential surface of its cylindrical wall, for engaging the externally threaded portion 24 formed on the outer circumferential surface of the receptacle 16 of the container body 12. The thus constructed cap member 56 is removably attached to the receptacle 16 by engagement of the internally threaded portion 26 with the externally threaded portion 24 of the receptacle 16 of the container body 12. In this embodiment, too, the recess 20 is fluid-tightly closed upon assembling of the cap member 56 with the receptacle 16.

Unlike the accommodating structure 32 as used in the above first embodiment, the accommodating structure 58 is a member separate from the cap member 56, and has a generally dish-like shape consisting of a circular bottom wall and a frustoconical wall. In this accommodating structure 58, a cylindrical partition wall 60 having a relatively small height is formed so as to extend from the central portion of the bottom wall. The inner space defined by the partition wall 60 serves as an accommodating chamber 62 for accommodating the oxidizing treating liquid 42 as the first treating agent. The accommodating structure 58 has a pair of curved elongate communication holes 64, 64. The communication holes 64, 64 are formed in the outer peripheral portion of the bottom wall, so as to extend along the circumference of the partition wall 60. The accommodating structure 58 further has an outward flange 66 which extends radially outwardly from the periphery of the upper end portion of the frustoconical wall. This outward flange 66 is formed integrally with the frustoconical wall, so as to continuously extend in the circumferential direction.

The accommodating structure 58 is superposed on the receptacle 16 of the container body 12, such that the outward flange 66 formed integrally with the frustoconical wall is placed on the upper end face of the receptacle 16, so that the two communication holes 64, 64 formed in the bottom wall of the accommodating structure 58 and the base portion of the partition wall 60 defining the accommodating chamber 62 are submerged within the reducing agent 40 which is accommodated in the recess 20 of the container body 12, together with the contact lens 38. Further, the cap member 56 is attached to the receptacle 16 on which the accommodating structure 58 is superposed as described above, with the outward flange 66 of the accommodating structure 58 being sandwiched by and between the cap member 56 and the upper end face of the receptacle 16 of the container body 12, whereby the accommodating structure 58 is disposed in the fluid-tight space in the recess 20 of the container body 12. Since the accommodating structure 58 of this embodiment is formed of a soft resin material, and the outward flange 66 sandwiched by and between the cap member 56 and the upper end face of the receptacle 16 of the container body 12 functions as a sealing member for improving the fluid-tightness of the fluid-tight space defined upon closure of the recess 20 by the cap member 56.

While the thus constructed treating container 54 is kept stationary with the recess 20 of the container body 12 being fluid-tightly closed, the reducing agent 40 in the recess 20 is allowed to flow into the accommodating structure 58 through the two communication holes 64, 64, and is inhibited from flowing into the accommodating chamber 62 of the accommodating structure, in which the oxidizing treating liquid 42 is accommodated. The container body 12 is shaken frontwards and rearwards, leftwards and rightwards, or upwards and downwards, whereby the oxidizing treating liquid 62 in the accommodating chamber 62 flows therefrom over the partition wall 60, and is mixed with the reducing agent 40 which has flowed into the accommodating structure 58, while at the same time flowing into the recess 20 through the two communication holes 64, 64. Further, the reducing treating liquid 40 which has flowed into the accommodating structure 58 flows into the accommodating chamber 62 over the partition wall 60, and is mixed with the oxidizing treating liquid 42 in the chamber 62. The mixture of the treating agents then flows from the chamber 62 into the recess 20 through the two communication holes 64, 64, and is mixed with the reducing treating liquid 40 in the recess 20.

In the treating container 54 constructed as described above, the contact lens 38 can be disinfected by contact with the iodine in the oxidizing treating liquid 42, such that the reducing treating liquid 40, the oxidizing treating liquid 42 and the contact lens immersed in the reducing treating liquid 40 are prevented from being exposed to the external air. At the same time, the iodine is made colorless and transparent by the reducing activity of the EDTA in the reducing treating liquid 40.

The thus constructed treating container 54 of the present embodiment enjoys the advantages as provided by the treating container in the above embodiments that the contact lens 38 can be sufficiently disinfected while it is prevented from being colored.

In the treating container 54 described above wherein the accommodating structure 58 is a member separate from the cap member 56, there is no need to form an inlet in the cap member 56 for introducing the treating liquid into the accommodating structure 58, and to provide a closure member for closing the inlet, as in the treating containers of Figs. 3 and 4. Therefore, the number of the required components can be reduced, resulting in a simplified structure of the treating container.

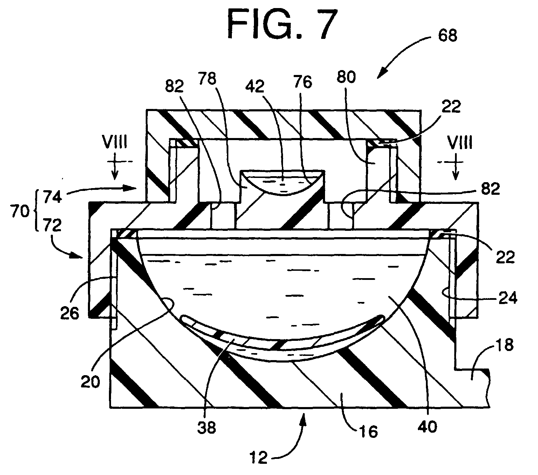

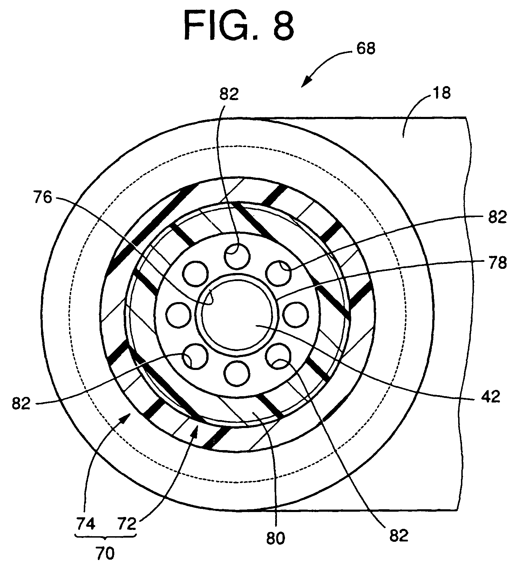

Referring next to Figs. 7 and 8, there is shown a treating container according to another embodiment, which has an accommodating structure and a cap member different from those of the treating containers of the above embodiments. The treating container of this embodiment has a cap member 70 consisting of a lower cap 72 and an upper cap 74.

The lower cap 72 of the cap member 70 is a generally cylindrical member closed at one of its opposite ends. The bottom wall of the lower cap 72 has a size large enough to close the opening of the receptacle 16 of the container body 12. The lower cap 72 has a protrusion 78 which protrudes from a central portion of the bottom wall by a suitable axial distance. In the upper axial end of the central protrusion 78, there is formed a recess 76 having a semi-spherical bottom surface. Radially outwardly of the central protrusion 78, a cylindrical portion 80 is formed to surround the protrusion 78. This cylindrical portion 80 has a height or axial dimension larger than the protrusion 78 and includes an externally threaded portion formed on its outer circumferential surface. The lower cap 72 further includes a plurality of through-holes 82 (eight through-holes in this embodiment) formed through the thickness of the bottom wall, radially intermediate between the protrusion 78 and the cylindrical portion 80, such that the through-holes 82 are spaced apart from each other by a suitable distance in the circumferential direction of the lower cap 72.

The upper cap 74 is a generally cylindrical member closed at one of its opposite ends, and includes an internally threaded portion formed on its inner circumferential surface. The bottom wall of the upper cap 74 has a size large enough to close the opening of the cylindrical portion 80 of the lower cap 72.

The lower cap 72 which accommodates the iodine-containing oxidizing treating liquid 42 in the recess 76 of the protrusion 78 is removably attached to the receptacle 16 of the container body 12, such that the internally threaded portion 26 formed on the inner circumferential surface of the lower cap 72 engages the externally threaded portion of 24 the receptacle 16. The upper cap 74 is removably attached to the lower cap 72 by engagement of its internally threaded portion with the externally threaded portion of the cylindrical portion 80. According to this arrangement, the opening of the recess 20 of the receptacle 16 is closed by the cap member 70, so that the recess 20 is fluid-tightly closed while it is held in communication with the inner space of the cylindrical portion 80 of the lower cap 72 through the plurality of through-holes 28 formed in the lower cap 72.

In the thus constructed treating container 68, the container body 12 is oscillated or shaken frontwards and rearwards, leftwards and rightwards, or upwards and downwards while the recess 20 of the container body 12, in which the reducing agent 40 is accommodated with the contact lens 38 being immersed therein, is closed by the cap member 70, whereby the oxidizing treating liquid 42 in the accommodating chamber 33 and the reducing treating liquid 40 in the recess 20 are mixed with each other through the through-holes 82 of the lower cap 72.

In the present treating container 68, the cylindrical portion 80 formed on the bottom wall of the lower cap 72 and having the inner space provides the accommodating structure while the recess 76 of the protrusion 78 formed on the bottom wall of the lower cap 72 and the plurality of through-holes 82 formed through the the bottom wall function as the accommodating chamber and the communication means, respectively. While the accommodating structure is formed integrally with the lower cap 72 in the present embodiment, the accommodating structure may be separate from the lower cap 72. Namely, the accommodating structure which has the protrusion 78 having the recess 76 as the accommodating chamber and the plurality of through-holes 82 formed around the protrusion 76 as the communication means may be disposed within the cylindrical portion 80 of the lower cap 72, such that it is retained at a portion sandwiched by and between the upper end face of the cylindrical portion 80 and the inner surface of the bottom wall of the upper cap 74, in the manner similar to that of the above second embodiment.

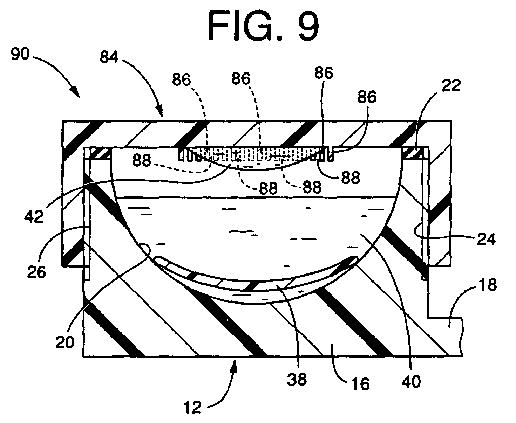

In the treating containers of the above-described embodiments, the second treating agent is accommodated in the accommodated structure. Fig. 9 shows a treating container according to another embodiment, wherein the second treating agent is retained by special retaining means.

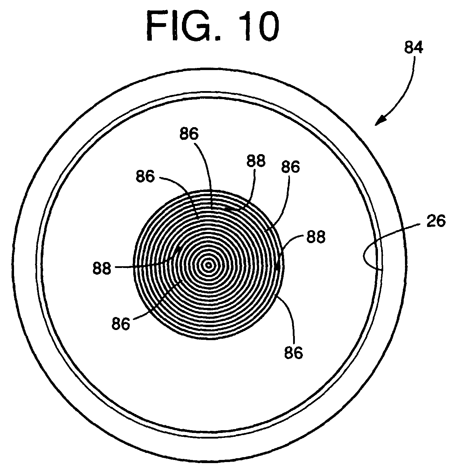

Namely, a plurality of projections 86 as the retaining means are formed integrally with a cap member 84 which is closed at one of its opposite ends, such that the projections 86 extend from the central part of the inner surface of the bottom wall of the cap member 84. As is apparent from Fig. 10, each of the plurality of projections 86 is in the form of an annular rib having a small wall thickness. These annular ribs have respective different diameters, and are formed concentrically with one another on the inner surface of the bottom wall of the cap member 84, such that the ribs are spaced apart from each other by a considerably small distance. Between the adjacent ones of the thus formed plurality of projections 86, a minute annular gap 88 is formed. According to this arrangement, a plurality of gaps 88 are concentrically formed corresponding to the plurality of projections 86.

In the thus constructed treating container 90, the oxidizing treating liquid 42 adheres to the wall of each projection 86 based on surface tension, and is retained in the plurality of gaps 88 against gravity, as shown in Fig. 9.

In the treating container 90 of this embodiment, too, the oxidizing treating liquid 42 retained in the gaps 88 defined by the plurality of projections 86 is prevented from mixing with the reducing treating liquid accommodated in the recess 20 of the receptacle 16 of the container body 12 with the contact lens 38 being immersed therein, as long as the container body 12 whose recess 20 is fluid-tightly closed by the cap member 84 is kept stationary. By shaking the container body 12 frontwards and rearwards, leftwards and rightwards, or upwards and downwards, the oxidizing treating liquid 42 and the reducing treating liquid 40 are freely mixed with each other.

In the thus constructed treating container 90, the retaining means for retaining the oxidizing treating liquid 42 is provided simply by forming the plurality of projections 86 defining the plurality of gaps 88 therebetween, on the inner surface of the bottom wall of the cap member 84. Since the projections 86 and the gaps 88 are concentrically formed with one another, the oxidizing treating liquid 42 is retained with high stability.

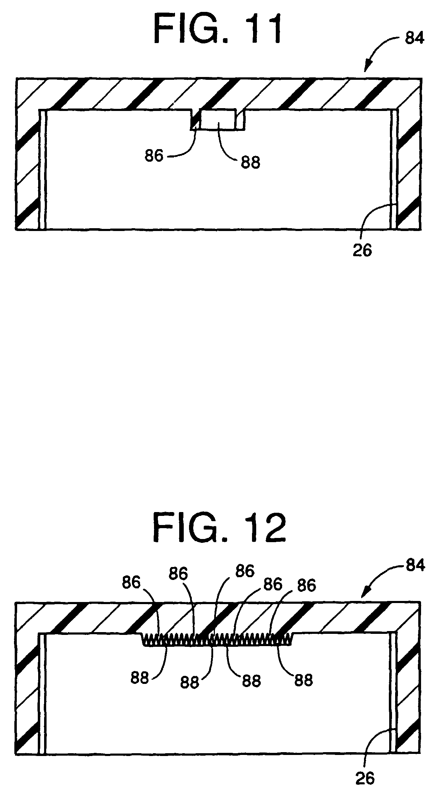

The number of the projections 86 as the retaining means is not limited to that of the present embodiment. As shown in Fig. 11, a single projection 86 having a cylindrical shape may be formed, and the inner bore of the single projection 86 provides the above-described gap 88.

The configuration of the projections 86 is not particularly limited. For instance, each of the plurality of projections 86 may have a triangular cross sectional shape as shown in Fig. 12. Alternatively, an entire array of the projections 86 may be block-shaped with a large wall thickness.

Further, the projections 86 may be formed parallel with each other on the inner surface of the bottom wall or the side wall of the cap member 84.

The arrangement of the projections 86 is not particularly limited. For instance, the cap member 84 may have a plurality of grooves formed in its inner surface. In this case, the projections 86 are provided by the walls defining the grooves, and the gap 88 is provided by the inner space of each groove.

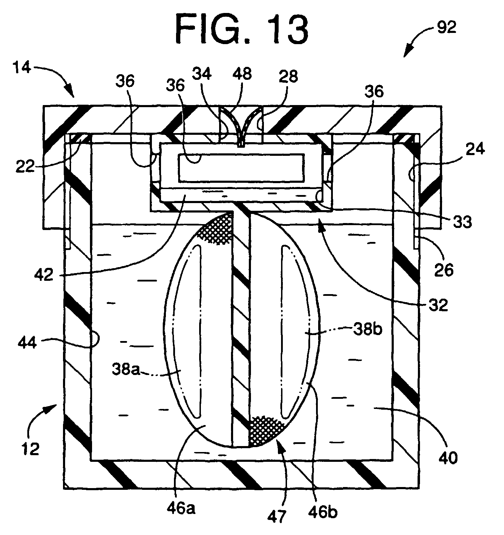

Referring next to Figs. 13-15, there are shown treating containers constructed according to further embodiments, wherein the container body provides a housing chamber in which a pair of contact lenses for a left and a right eye are stored together.

In a treating container 92 shown in Fig. 13, the container body 12, which is a cylindrical member closed at one of its opposite ends, has an inner space which provides an accommodating chamber 44 having a size large enough to store a pair of contact lens 38a and 38b for the left and right eyes, respectively, together with the reducing treating liquid 40. To the inner surface of the cap member 14, the accommodating structure 32 is fixed. To this accommodating structure 32, there is attached a lens holder 47 including two baskets 46a, 46b for respectively accommodating the contact lens 38a for the left eye and the contact lens 38b for the right eye. The baskets 46a, 46b are formed of nets which permit the reducing treating liquid 40, the oxidizing treating liquid 42 and the mixture thereof to pass therethrough.

The container body 12 is covered by the cap member 14 with the contact lenses 38 being respectively accommodated within the two baskets 46a, 46b of the lens holder 47. Thus, the contact lenses 38a, 38b for the left and right eyes are accommodated together in the accommodating chamber 44 of the container body 12.

In the present embodiment, the container body 12 is a simple cylindrical member having only one opening, so that its structure is simplified. Further, the present arrangement needs only one cap member 14, to thereby effectively reduce the number of the required components of the treating container.

The mechanism for attaching the lens holder 47 to the accommodating structure 32, and the mechanism for bringing the contact lenses 38 into and out of the lens holder 47 are similar to those disclosed in the Publication No. 7-48093 of the examined Japanese Utility Model Application.

In the above first embodiment, the through-hole 28 formed in the cap member 14 as an inlet for introducing the treating liquid into the accommodation structure 32 is fluid-tightly closed by the plug 30 which is removably attached to the cap member 14. In place of such a plug 30, the cap member 14 of this embodiment has a check valve 48 provided in the through-hole 28. This check valve 48 has a relatively simple structure having a duckbill shape, for instance. This arrangement does not require the step of attaching and removing the plug 30 to and from the cap member 14 in introducing the oxidizing treating liquid 42 into the accommodating structure 32, advantageously simplifying the operation of introducing the oxidizing treating liquid 42 into the accommodating structure 32. The oxidizing treating liquid 42 may be introduced into the accommodating structure 32 through the four windows 36 formed therein. In this case, the communication hole 34 of the accommodating structure 32 and the through-hole 28 of the cap member 14 are not required.

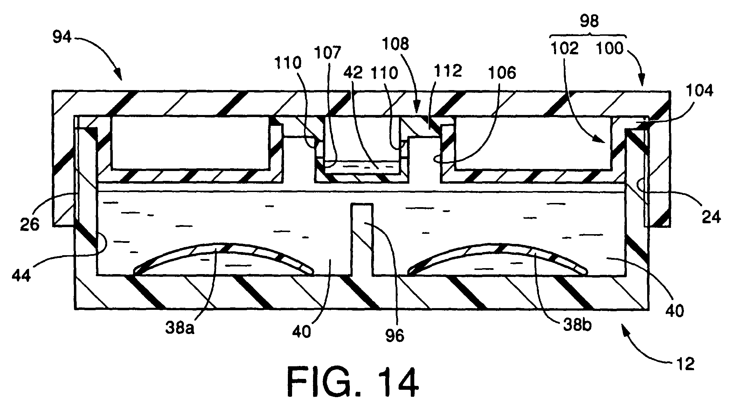

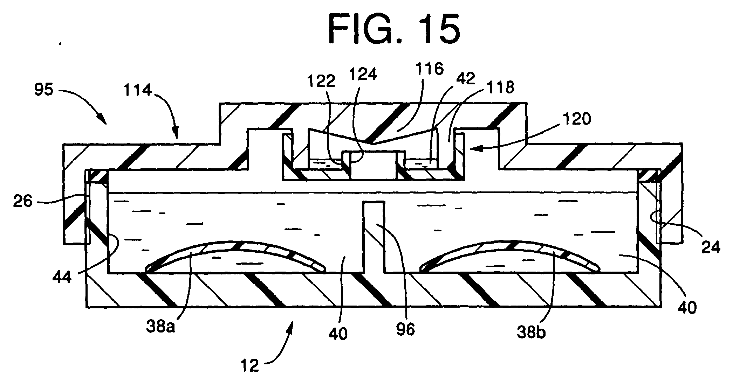

In treating containers 94, 95 shown in Figs. 14 and 15, respectively, the container body 12, which is a cylindrical member closed at one of its opposite ends, has the inner space serving as the accommodating chamber 44 having one opening through which the accommodating chamber 44 is open to the outside. The inner space of this accommodating chamber 44 is divided into two sections by a partition wall 96 having a height smaller than the side wall of the container body 12 and extending upright from its bottom wall. In the thus formed two sections, a pair of contact lenses 38a, 38b are respectively accommodated, together with the reducing treating liquid 40.

The treating container 94 shown in Fig. 14 has a cap member 98 consisting of an outer cap 100 and an inner cap 102. The outer cap 100 removably engages the container body 12, so that the opening of the container body 12 is closed for fluid-tightly sealing the accommodating chamber 44. The inner cap 102 of the cap member 98 has a hole 106 formed in its central portion, and a flange 104 integrally formed at its outer peripheral portion. The thus formed inner cap 102 is sandwiched by and between the container body 12 and the outer cap 100 at its flange 104 with the accommodating chamber 44 being fluid-tightly closed by the outer cap 100, so that the inner cap 102 is located above the liquid surface of the reducing treating liquid 40 in the accommodating chamber 44. In the treating container 94 wherein the accommodating chamber 44 is fluid-tightly closed as described above, an accommodating structure 108 is fitted in the hole 106 of the inner cap 102. This accommodating structure 108, which is a cylindrical member closed at one of its opposite ends, has an inner space serving as an accommodating chamber 107, and a plurality of windows 110 formed through the lower portion of its side wall.

The accommodating structure 108 whose accommodating chamber 107 stores the oxidizing treating liquid 42 is sandwiched by and between the outer cap 100 and the inner cap 102 at its outward flange 112 with the plurality of windows 110 being open to the accommodating chamber 44 in which the reducing treating liquid 40 is accommodated.

The treating container 95 shown in Fig. 15 includes a cap member 114 having a tapered portion 116 which protrudes from the central part of the inner surface of its bottom wall in the downward direction by a suitable distance. Around the tapered portion 116, there is formed a cylindrical protrusion 118 so as to surround the tapered portion 116. To this cylindrical protrusion 118, there is attached an accommodating structure 120 which has an integral structure consisting of an annular bottom wall, an inner cylindrical portion with a small diameter formed at a radially inner portion of the bottom wall, and an outer cylindrical portion with a large diameter formed at a radially outer portion of the bottom wall. In this arrangement, an accommodating chamber 122 is provided by the space defined by the two cylindrical portions and the bottom wall, while a communication hole 124 is provided by an inner hole of the small-diameter inner cylindrical portion.

The cap member 114 is attached to the container body 12 so as to close its opening, so that the accommodating chamber 44 of the container body 12 is fluid-tightly closed while the accommodating structure 12, which accommodates the oxidizing treating liquid 42 in the accommodating chamber 122, is located in the fluid-tight space of the container body 12 with the communication hole 124 being open to the space above the liquid surface of the reducing agent 40 in the housing chamber 44.

In each of the treating containers 94, 95, the container body 12 is shaken frontwards and rearwards, leftwards and rightwards, or upwards and downwards with the accommodating chamber 44 being fluid-tightly closed by the cap member 98, 114, whereby the oxidizing treating liquid 42 and the reducing treating liquid 40 are mixed with each other.

For introducing the oxidizing treating liquid 42 into the accommodating chamber 122 of the accommodating structure 120 in the treating container 95 of Fig. 15, the cap member 114 is turned inside up, and the oxidizing treating liquid 42 is poured into the interior of the cylindrical protrusion 118, so that the oxidizing treating liquid 42 flows along the tapered surface of the tapered portion 116, and is accordingly accumulated in the bottom of the tapered portion 116. Thereafter, the cap member 114 is turned over, so that the oxidizing treating liquid 42 flows into the accommodating chamber 122 of the accommodating structure 120.

While various configurations of the accommodating structure are shown in the above-illustrated embodiments, the configuration of the accommodating structure is not limited to those in the illustrated embodiments. The accommodating structure may be shaped otherwise as long as it accommodates a suitable treating agent therein.

It is needless to mention that the configuration, number and position of the windows or the communication holes formed in the accommodating structure are not limited to those in the illustrated embodiments.

The kind of the first treating agent accommodated in the container body, and the kind of the second treating agent accommodated in the accommodating structure or retained by the retaining means are not limited to those in the above embodiments. The first treating agent may be selected from among various liquid treating agents (including the ones obtained by dissolving solid agents) in which the contact lens is immersed. The second treating agent may be selected from among various liquid treating agents (including the ones obtained by dissolving solid agent) and solid treating agents (including tablet, granule and powder forms), which are mixed with the first treating agent. The first and second treating agents are suitably selected and used in combination, depending upon the kind of treatment of the contact lens and the method of the treatment, by taking account of the influence on the contact lens.

For disinfecting the contact lens, for instance, the reducing agent which contains citric acid, ascorbic acid, sorbic acid, thiosulfuric acid, sulfurous acid or a salt thereof or hydrogen sulfite is used as the first treating agent, in addition to the reducing treating liquid containing the EDTA or its salt. As the second treating agent, the oxidizing treating liquid or solid oxidizing treating agent which contains oxyacids of halogen such as hypochlorous acid, chlorous acid and hypobromous acid or a salt thereof is used, in addition to the oxidizing treating liquid containing the iodine. The oxidizing treating agent containing the oxyacid of halogen or its salt which gives a small effect on the contact lens can be used as the first treating agent while the above-described various reducing treating liquids can be used as the second treating agent. The reducing treating liquid which contains, as the sulfur-containing reducing agent, thiosulfuric acid, sulfurous acid or a salt thereof or hydrogen sulfite can be used as the second treating agent in view of its high reducing activity, while the iodine-containing oxidizing treating agent can be used as the first treating agent.

When the contact lens is cleaned subsequently after it has been disinfected, the disinfecting liquid containing disinfectants known in the art such as PHMB and chlorhexidine gluconate is used as the first treating agent while the liquid or solid cleaning agent containing protease or lipase is used as the second treating agent.

In the above embodiments, suitably selected pH buffer and tonicity agent are contained in the reducing treating liquid containing the EDTA or its salt used as the first treating agent. Since the reducing treating liquid is mixed with the iodine-containing oxidizing treating liquid as the second treating agent for disinfection of the contact lens, such pH buffer and tonicity agent may be included in the oxidizing treating liquid. In other words, the pH buffer and the tonicity agent are included in at least one of the reducing treating liquid and the oxidizing treating liquid. The kinds of the pH buffer and the tonicity agent are not particularly limited, and any known pH buffers and tonicity agents are suitably used. For instance, a phosphate buffer, a borate buffer, or a citrate buffer is used as the pH buffer, while an ionic tonicity agent such as sodium chloride, potassium chloride or sodium sulfate, or a nonionic tonicity agent such as glycerin, propyleneglycol or polyethtleneglycon is used.

While the container bodies and the cap members of the above embodiments and the plug used in the first embodiment are formed of a transparent resin material, the material of these members is not particularly limited, and may be suitably selected from among known materials conventionally used for contact lens treating containers and accommodating cases. Where the reducing treating liquid containing the EDTA or its salt and used as the first treating agent, and the oxidizing agent containing the iodine and used as the second treating agent are mixed with each other in the container body, at least the container body is preferably formed of a transparent resin material for effectively making the iodine in the oxidizing treating liquid colorless and transparent by the the EDTA. As the transparent resin material, it is preferable to employ a widely used resin material such as polycarbonate, polymethyl methacrylate, polyethylene, polypropylene or polystyrene.

In the above-described two embodiments, the present invention was applied to the treating containers used for disinfecting the contact lens by using the suitable oxidizing treating agent and reducing treating agent. It is needless to mention that the present invention is applicable to any treating containers used for effecting a desired treatment of the contact lens by using two different treating agents, wherein the contact lens is immersed in one of the treating agents in a liquid form, and the other treating agent is added for mixture with the one treating agent.

While the present invention has been described in detail in its specific embodiments, it is needless to mention that the invention is not limited to the details of the illustrated embodiments. It is to be understood that the present invention may be otherwise embodied with various changes, modifications and improvements, which may occur to those skilled in the art, without departing from the scope of the present invention.

When the contact lens is subjected to a suitable treatment in the contact lens treating container of the present invention by using two different treating agents, the contact lens is immersed in one of the two treating agents which is a liquid, and the other treating agent is added thereto so that the two treating agents are mixed with each other, without suffering from various troubles caused by exposure of the contact lens, the two treating agents and the inside of the container body to the external air, since the other treating agent is added to and mixed with the one treating agent with the container body being fluid-tightly closed. Accordingly, the contact lens can be sufficiently and smoothly treated with the two treating agents. Further, the present invention is also characterized in that the the two treating agents can be quickly mixed with each other.

### Industrial Utility

As is clear from the above explanations, the present invention relates to an improvement of the contact lens treating container used for treating the contact lens with two different treating agents, wherein the contact lens can be treated effectively and quickly, without suffering from various troubles caused by contact of the contact lens, the two liquid agents and the inside of the container body with the external air.

## Claims

1. A treating container for a contact lens characterized by comprising:
a container body accommodating a liquid first treating agent in which said contact lens to be treated is immersed, and having an upper opening through which said contact lens is brought into and out of said container body;
a cap member which fluid-tightly closes said opening of said container body; and
retaining means which is disposed in a fluid-tight space defined upon closure of said opening of said container body by said cap member, said retaining means retaining a second treating agent for treating said contact lens, such that said second treating agent is inhibited from freely mixing with said first treating agent while said container body is kept stationary, and such that said first treating agent in said container body and said second treating agent retained by said retaining means can be freely mixed with each other when said container body is shaken while said opening is closed by said cap member.

2. A treating container according to claim 1, characterized in that said retaining means is constituted by at least one projection which protrudes from an inner surface of said cap member so as to form a minute gap, said second treating agent being retained in said minute gap against gravity.

3. A treating container according to claim 2, characterized in that said at least one projection comprises a plurality of concentric annular projecting ribs which protrude from said inner surface of said cap member, said minute gap being formed between adjacent ones of said plurality of projecting ribs.

4. A treating container for a contact lens characterized by comprising:
a container body accommodating a liquid first treating agent in which said contact lens to be treated is immersed, and having an upper opening through which said contact lens is brought into and out of said container body;
a cap member which fluid-tightly closes said opening of said container body; and
an accommodating structure disposed in a fluid-tight space defined upon closure of said opening of said container body by said cap member, and having an accommodating chamber for accommodating therein a second treating agent for treating said contact lens, and a communication hole which permits a flow of said first treating agent into said accommodating chamber, said first and said second treating agents being inhibited from freely mixing with each other while said container body is kept stationary, and such that said first treating agent in said container body and said second treating agent in said accommodating structure can be fed through said communication hole when said container body is shaken while said opening is closed by said cap member.

5. A treating container according to claim 4, characterized in that said accommodating structure is disposed in said fluid-tight space with a portion thereof being immersed in said first treating agent, said first treating agent flowing into said accommodating structure through said communication hole which is formed in said portion of said accommodating structure immersed in said first treating agent, said accommodating chamber of said accommodating structure being defined by a partition wall whose upper end is located above a level of said first treating agent which has flowed into said accommodating structure, so that said first treating agent is inhibited from flowing into said accommodating chamber while said container body is kept stationary.

6. A treating container for a contact lens characterized by comprising:
a container body accommodating a liquid first treating agent in which said contact lens to be treated is immersed, and having an upper opening through which said contact lens is brought into and out of said container body;
a cap member which fluid-tightly closes said opening of said container body; and
an accommodating structure disposed in a fluid-tight space defined upon closure of said opening of said container body by said cap member, and having an accommodating chamber for accommodating therein a second treating agent for treating said contact lens, and a window which is open to a portion of said fluid-tight space which is located above a level of said first treating agent accommodated in said container body, and such that said first treating agent in said container body and said second treating agent in said accommodating structure can be mixed with each other through said window when said container body is shaken while said opening is closed by said cap member,

7. A treating container according to any one of claims 4, 5 and 6, characterized in that said accommodating structure is fixed to an inner surface of said cap member which defines said fluid-tight space, said accommodating structure being disposed in said fluid-tight space when said container body is closed by said cap member.

8. A treating container according to any one of claims 4, 5 and 6, characterized in that said accommodation structure is a member separate from said cap member which defines said enclosed space, and has a flange which is sandwiched by and between said cap member and said container body, said accommodating structure being disposed in said fluid-tight space when said container body is closed by said cap member, with said flange being sandwiched by and between said container body and said cap member.

9. A treating container according to any one of claims 1-8, characterized in that said container body includes an accommodating chamber having one opening and storing a pair of contact lenses for a left and a right eye, said pair of contact lenses being brought into and out of said accommodating chamber through said opening.

10. A treating container according to any one of claims 1-9, characterized in that one of said first and second treating agents is an iodine disinfectant, while the other is a reducing agent which makes said iodine disinfectant colorless and transparent when said reducing agent is mixed with said iodine disinfectant.

11. A treating container according to claim 10, characterized in that said container body is formed of a transparent resin material.
